# EUROPÄISCHE PATENTSCHRIFT

(11) **EP 0 706 650 B1**
(45) Veröffentlichungstag und Bekanntmachung des Hinweises auf die Patenterteilung: **29.04.1998**
(21) Anmeldenummer: 94918739.7
(22) Anmeldetag: 24.06.1994
(51) Int. Cl.: G01N 23/227, G01N 15/06, G01N 27/00

(54) **VERFAHREN UND VORRICHTUNG ZUR QUANTITATIVEN BESTIMMUNG VON IN EINER PROBE BEFINDLICHEM MATERIAL**
METHOD FOR THE QUANTITATIVE DETERMINATION OF MATERIAL IN A SAMPLE
PROCEDE ET DISPOSITIF DE DETERMINATION QUANTITATIVE D'UN MATERIAU SITUE DANS UN ECHANTILLON

(30) Priorität: 29.06.1993 DE 4321456
(43) Veröffentlichungstag der Anmeldung: 17.04.1996
(73) Patentinhaber: FORSCHUNGSZENTRUM JÜLICH GMBH, 52425 Jülich (DE)
(72) Erfinder: HACKFORT, Helmut, D-50858 Köln (DE); HINZEN, Georg, D-52074 Aachen (DE)
(86) Internationale Anmeldenummer: DE9400731
(87) Internationale Veröffentlichungsnummer: WO9501563

(56) Entgegenhaltungen:
- EP-A- 0 147 521
- EP-A- 0 160 888
- EP-A- 0 163 291
- DE-A- 3 422 053
- DE-A- 3 422 054
- JOURNAL OF APPLIED PHYSICS, Bd.55, Nr.5, März 1984, NEW YORK US Seiten 1388 - 1393 'X-RAY PHOTOEMISSION OF POLYNUCLEAR AROMATIC CARBON'

## Beschreibung

Die Erfindung bezieht sich auf ein Verfahren und eine Vorrichtung zur quantitativen Bestimmung von in einer Probe befindlichem Material.

Ein derartiges Verfahren oder ein Meßgerät wäre in vielen Anwendungsfällen einzusetzen, beispielsweise für die quantitative Bestimmung des Kohlegehalts eines für die Verbrennung vorgesehenen Brennstoffs und für die qualitative Bestimmung des organischen Anteils in einem Lebensmittel oder einer anderen Probe.

Aus der DE 34 22 054 C2 ist bekannt, mittels Photoemissionsmessung für in Gasen vorhandene Schwebeteilchen oberflächenspezifische Informationen zu erhalten. Dies geschieht durch quantitative Bestimmung von Stoffen, die bei einer bestimmten Temperatur von den Partikeln abdampfen. Untersucht werden u. a. Aerosole aus Verbrennungen organischen Materials. Hieraus ist offenbart, daß diese eine hohe photoelektrische Aktivität besitzen, die durch Substanzen auf ihren Oberflächen verursacht wird. Bei diesem Verfahren wird das im Photoemissionsteil der Apparatur gemessene Signal als Maß für die auf der Aerosoloberfläche abgeschiedene Materialmenge verwendet.

Meßergebnisse werden mittels der photoelektrischen Aktivität ermittelt.

Aus EP-A-0147521 Verfahren zur Regelung von Verbrennungsprozessen bekannt. Das Verfahren bezieht sich nur auf die auf einer Probe befindlichen adsorbierten Stoffe.

Dem vorgenannten Stand der Technik gegenüber ist es Aufgabe der Erfindung, ein Verfahren zu schaffen, das es ermöglicht, den Anteil des Materials, aus dem die Probe besteht, zu bestimmen. Die Aufgabe der Erfindung bezieht sich ferner auf eine Vorrichtung zur Durchführung des Verfahrens.

Diese Aufgabe wird erfindungsgemäß durch ein Verfahren mit den Merkmalen des Anspruchs 1 gelöst.

In Untersuchungen konnte gezeigt werden (siehe die Erläuterung zu den Figuren), daß zwischen dem mittels eines photoelektrischen Aerosolsensors erhaltenen Signal und dem Anteil an Material in der Probe eine direkte Beziehung besteht, vorausgesetzt, es handelt sich um Material, das als Submikronteilchen mit einem photoemissionsfähigen Stoff bedeckt ist.

Um das Material der Probe in einen photoemissionsfähigen Zustand zu bringen, kann es zweckmäßig sein, das Material vorab mechanisch zu behandeln, beispielsweise zu mahlen. Dabei kann es außerdem angebracht sein, der Probe vor oder während der Behandlung zur Erzeugung des photoemissionsfähigen Zustandes zur Verdünnung einen nichtemissionsfähigen, inerten Feststoff in geeigneter Form zuzuführen.

Eine alternative Verfahrensweise besteht darin, daß der Probe vor oder während der Thermobehandlung inerter Feststoff als zusätzlicher Trägerstoff zugefügt wird. Dieser zusätzliche Trägerstoff dient dazu, den ggf. in Dampfform vorliegenden, das Signal bewirkenden Stoff als Adsorbat aufzunehmen und so der Messung zugänglich zu machen.

Ebenso kann es zweckmäßig sein, der Probe ein zusätzliches Trägergas, beispielsweise ein Inertgas, beizumischen.

Bei geeigneter Aufbereitung der Probe kann das Material der Photoemissionsmessung kontinuierlich zugeführt und ein entsprechend kontinuierliches Meßsignal erhalten werden.

Befinden sich in der Probe mehr als ein photoemissionsfähiges Material, dann wird die Probe oder ein Teil der Probe in unterschiedlichen photoemissionsfähigen Zuständen der Photoemissionsmessung zugeführt und bei der quantitativen Bestimmung der Materialien die Meßdaten entsprechender Vergleichsproben oder entsprechende, durch Kalibrierung ermittelte Daten berücksichtigt.

Für den Fall, daß das Material ein organisches Material ist und der die Submikronteilchen bedeckende photoemissionsfähige Stoff polyaromatische Kohlenwasserstoffe sind, ist es zweckmäßig, die Probe zur Erzielung des photoemissionsfähigen Zustandes auf eine Temperatur im Bereich von 600°C - 1000°C, vorzugsweise auf 750°C - 850°C zu erhitzen.

Das Verfahren zur Bestimmung des Anteils des Materials einer Probe kann auf verschiedene Weise in der Technik eingesetzt werden:

Bei kontinuierlicher Messung kann das Meßsignal als Führungsgröße zur Steuerung oder Regelung eines Verbrennungsprozesses eingesetzt werden.

Eine andere Anwendungsmöglichkeit des erfindungsgemäßen Verfahrens besteht darin, daß das Ergebnis der Bestimmung des Materials zur Überprüfung der Qualität der Probe (beispielsweise von Lebensmittelproben) eingesetzt wird.

Eine geeignete Vorrichtung zur quantitativen Bestimmung weist die Vorrichtungsmerkmale gemäß Nebenanspruch auf. Die dabei dem Sensor vorgeschaltete Einheit kann, falls das Material dadurch in den photoemissionsfähigen Zustand gebracht wird, eine Einheit zur Thermobehandlung der Probe sein. Insbesondere für organisches Material sollte die Einheit zur Thermobehandlung auf eine vorgegebene Temperatur im Bereich zwischen 600°C und 1000°C einstellbar sein.

Entsprechend den vorher erwähnten Verfahrensweisen sind wahlweise der Einheit zur Thermobehandlung eine Einheit zur Einspeisung von nichtemissionsfähigem, inertem Feststoff vorgeschaltet und/oder eine Einheit zum Einführen von zusätzlichem Trägergas.

Für den Fall, daß die Vorrichtung zur kontinuierlichen Bestimmung des Materials eingesetzt werden soll, ist eine Einrichtung zur kontinuierlichen Zufuhr der Probe vorgesehen und die elektronische Einheit zur kontinuierlichen Abgabe des Meßsignals ausgelegt. Der elektronischen Einheit ist zweckmäßigerweise eine Steuer- oder Regeleinrichtung nachgeschaltet.

Durch die erfindungsgemäße Lehre steht für Proben aus brennbarem Material oder Material mit einem brennbaren Anteil eine schnelle und einfache Vorrichtung zur Verfügung.

Die Ergebnisse der Probenbestimmung bzw. -analyse können z.B. für Kohle bei Produktüberwachung, Gerätesteuerung und/oder Prozeßkontrolle bei Gewinnung, Aufbereitung, Handel und Kraftwerksnutzung eingesetzt werden. Die direkte Steuerung einzelner Kohlestaubbrenner ist aufgrund der zugeführten Kohlemenge bzw. Kohlequalität möglich. Hinzu kommt die Regelung des Verbrennungsprozesses unter Einbeziehung einer Abgasanalyse. Die Messung betrifft dabei:
- die Erzeugung und Erhitzung eines strömenden Probestromes, ggf. Kohlestaubstromes;
- durch thermische Behandlung entsteht ein Abgasaerosol mit einer Bedeckung von polyzyklischen aromatischen Kohlenwasserstoffen (PAK bzw. PAH), die vom organischen Gehalt der Probe, von der Temperatur (Höhe, Dauer, ...) sowie von der Zusammensetzung des Umgebungsgases abhängt und
- die PAH-Bedeckung des strömenden Abgasaerosols wird durch On-line-Aerosol-Photo-Emission gemessen.

Anhand der Zeichnung werden das erfindungsgemäße Verfahren sowie die Vorrichtung näher erläutert und es werden Untersuchungsergebnisse über die Bestimmung von Material in einer Probe wiedergegeben. Die bei den Untersuchungen benutzten Verfahrensschritte entsprechen im wesentlichen denjenigen des Meßverfahrens. Eine Auswerte- und Vergleichseinheit zur Erzeugung eines Steuersignals für einen Prozeß wurde bei den Untersuchungen nicht eingesetzt. Eine Probennahme aus einem Prozeß wurde simuliert.

### Verfahrensschritte:

A Entnahme einer Probe aus einem Prozeß oder Dispergieren einer Probe in Trägergas (Luft, N, O etc.)
   kontinuierlich oder
   diskontinuierlich,
   kontinuierliche / diskontinuierliche Weitergabe der Probe.
B Konditionieren der staubförmigen Probe durch Thermobehandlung (z.B. thermische Exposition, Flashverdampfung, Laser).
   B.1 Benutzung einer oder mehrerer Temperaturen.
   B.2 Konditionieren des submikronen Probenaerosols (z.B. Verdünnung, Zugabe eines inerten Zusatzaerosols, Trocknung).
C Analyse-Einheit (lieferbare Sensoreinheit)
   Elektrostatischer Filter zur Absorption bereits geladener Teilchen;
   UV-Bestrahlungseinheit;
   Elektrometer, Verstärker.
D Auswerte-Einheit.

Es zeigen:
- Figur 1: Darstellung des Verfahrens der Probenumwandlung durch Thermobehandlung und Messung am Submikronaerosol;
- Figur 2: schematische Darstellung der Vorrichtung zur Messung und Signalerzeugung;
- Figur 3: Darstellung des Prinzips der Aerosol-Photoemission (APE) und UV-Bestrahlungseinheit;
- Figur 4: PAS-Signalkurven einer quantitativen Messung;
- Figur 5: PAS-Signalkurven einer qualitativen Messung (PAS ≡ Photoelektrischer-Aerosol-Sensor);
- Figur 6: Vorrichtung für Untersuchungen zur Bestimmung von in einer Probe befindlichem Material.

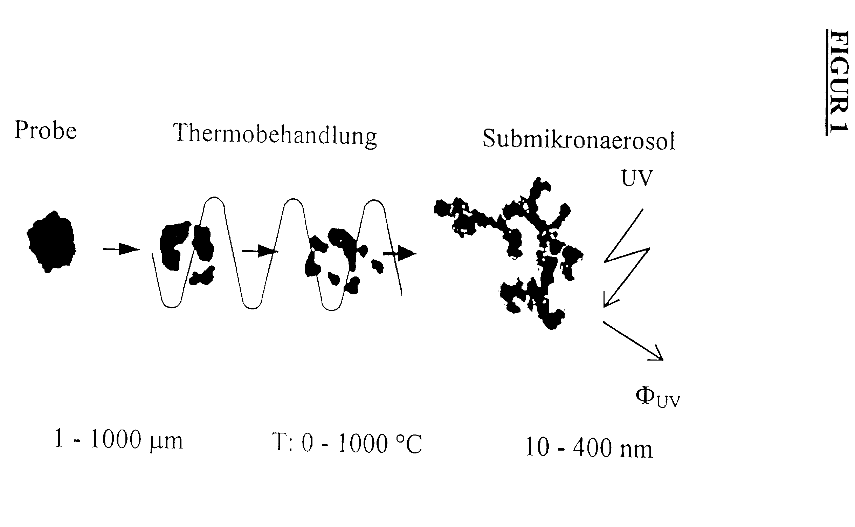

Figur 1 stellt schematisch die Problemumwandlung eines Staubkorns durch Thermobehandlung mit nachfolgender Messung am submikronen Verbrennungsaerosol dar. Der Durchmesser D_{I} eines Probenkornes liegt zunächst in der Größenordnung von 1 - 1000 µm. Bei der Thermobehandlung erfolgt eine Temperaturexposition bis ca. 1000°C. Es entsteht ein submikrones Verbrennungsaerosol mit Teilchengrößen D_{II} von etwa 10 - 400 nm. Eine Bestrahlung mit UV-Licht erzeugt einen Ionisationsstrom φ_{UV'} der u.a. von den Parametern T, D_{I} und D_{II} abhängig ist.

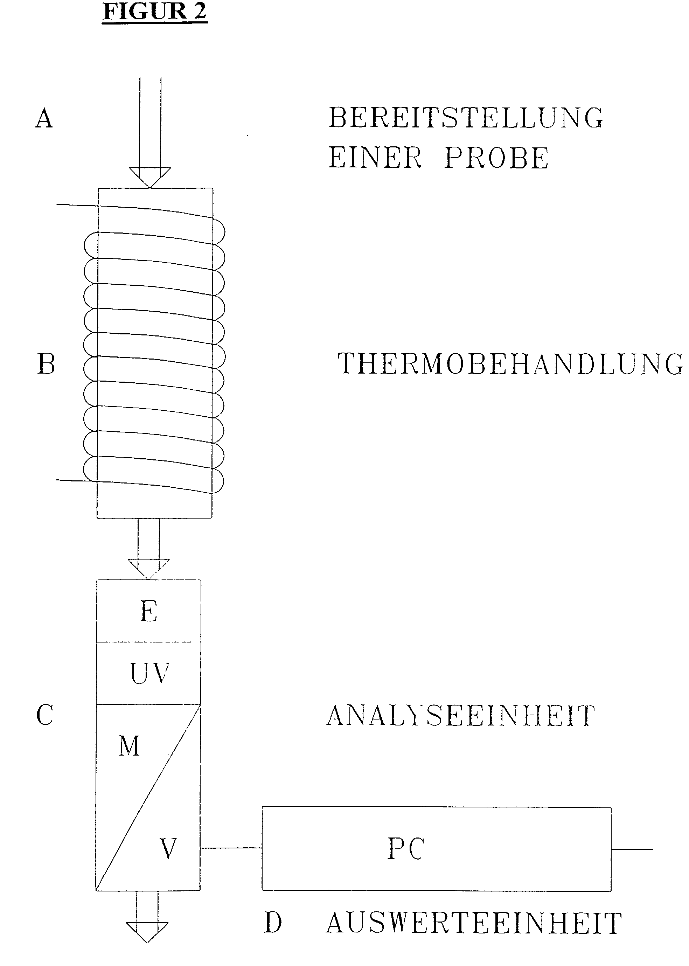

Figur 2 beschreibt die Verknüpfung der Bausteine einer Vorrichtung zur Bestimmung von in einer Probe befindlichem Material. Die Bereitstellung einer Probe (A) erfolgt durch direkte Entnahme aus einem Prozeß und/oder durch Verdünnung mit einem Trägermedium (z.B. Luft). Anschließend erfolgt eine Thermobehandlung (B) z.B. in einem Kapillarstrahlungsofen oder durch andersartige Erwärmung.

Der Analyse (C) durch Messung der Photoemission kann ein elektrostatischer Filter E vorgeschaltet werden. Notwendigerweise erfolgt eine Bestrahlung mit UV-Licht UV, eine Messung M der erzeugten Ladungsmenge und eine Verstärkung V des Meßsignals. In einer angeschlossenen Auswerteeinheit D, die z.B. ein PC sein kann, können z.B. eine Signalauswertung, ein Signalvergleich mit abgelegten Daten und die Erzeugung eines Steuersignals für einen Prozeß erfolgen.

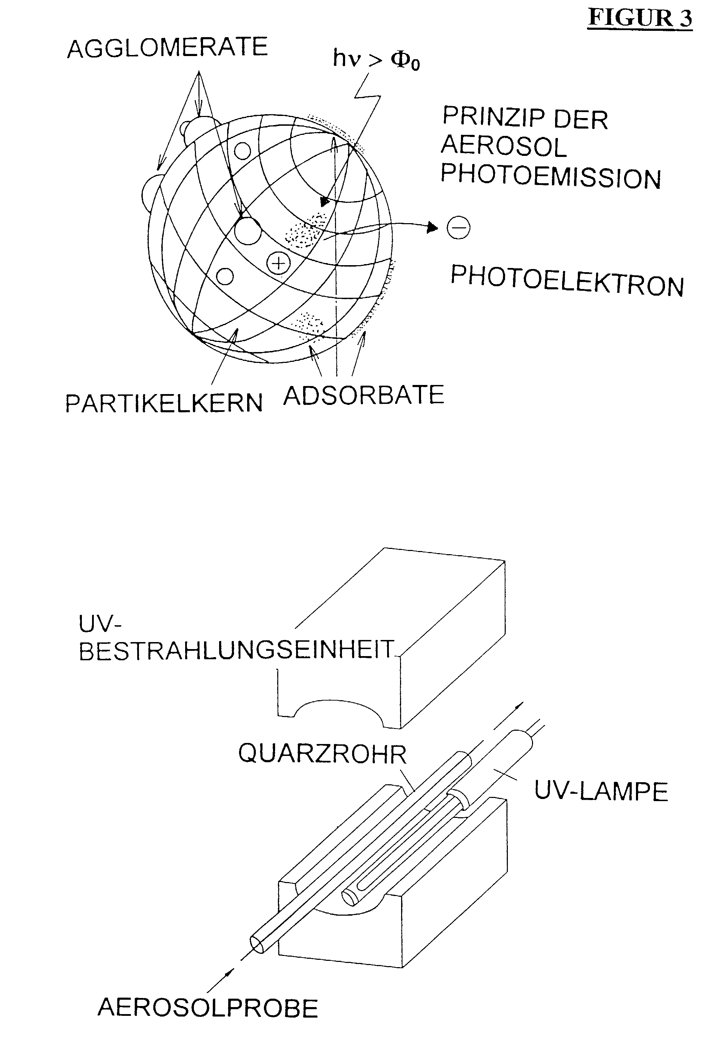

Figur 3 stellt zum einen das Modell eines submikronen, agglomerierten Teilchens dar, dessen Adsorbate durch UV-Bestrahlung zur Abgabe eines Photoelektrons angeregt wird (Prinzip der Aerosol-Photoemission, APE; hγ ≡ Lichtquantenergie, φ₀ Austrittsarbeit). Zum anderen ist in der dargestellten UV-Bestrahlungseinheit eine Anordnung gezeigt, bei der an einer fließenden Aerosolprobe eine kontinuierliche APE vorgenommen werden kann.

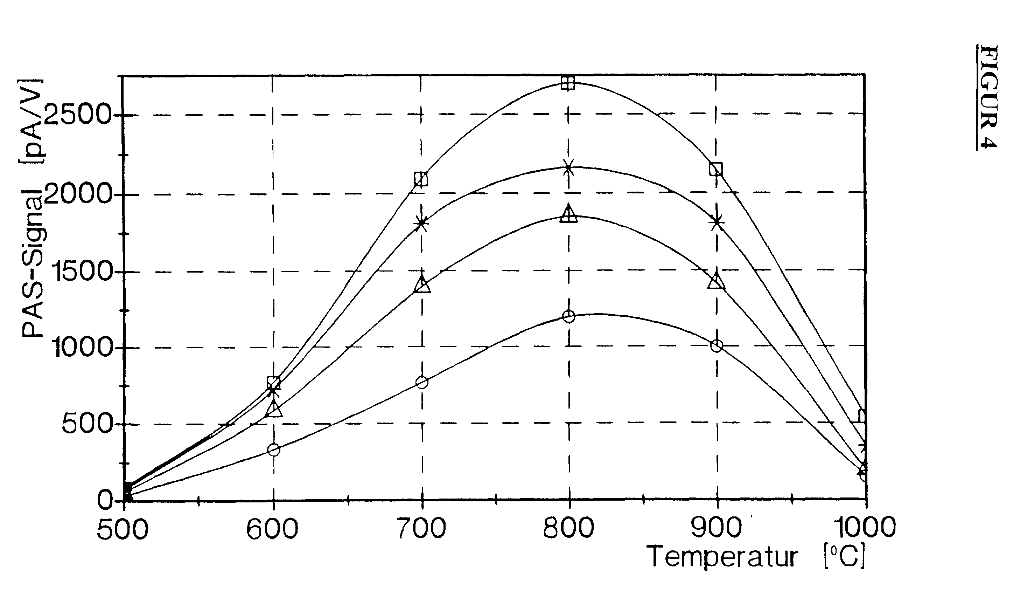

Figur 4 zeigt die Relation zwischen PAS-Signal, Temperatur und Kohlenstaub-Input. Wenn der Input reduziert wird, nimmt das PAS-Signal in ähnlicher Weise ab.

Das PAS-Signal nimmt ebenfalls ab, wenn der Anteil des Inertmaterials in der Probe ansteigt.

Das Maximum wird für verschiedene Kohlen bei den hier genutzten Parametern (insbesondere T, Lambda-Wert, Verweildauer, ...) bei einer Temperatur von etwa 800°C erreicht.

Bei dieser quantitativen Messung wurden als Proben 300, 225, 150 und 75 mg/h von einer Kohlenprobe bei 0,6 m³ Luft als Trägergas eingesetzt.

Aus dem Bereich der Lebensmittel wurde eine mehlprobe bei gleichen Anlagenparametern für verschiedene Massen (ca. 300, 225, 150, 75 mg/h; bei 0,6 m³ Tragluft pro Stunde) vermessen.

Die Signalkurven sind ähnlich denen für Kohle. Insbesondere liegt eine vergleichbare Massen- und Temperaturabhängigkeit vor.

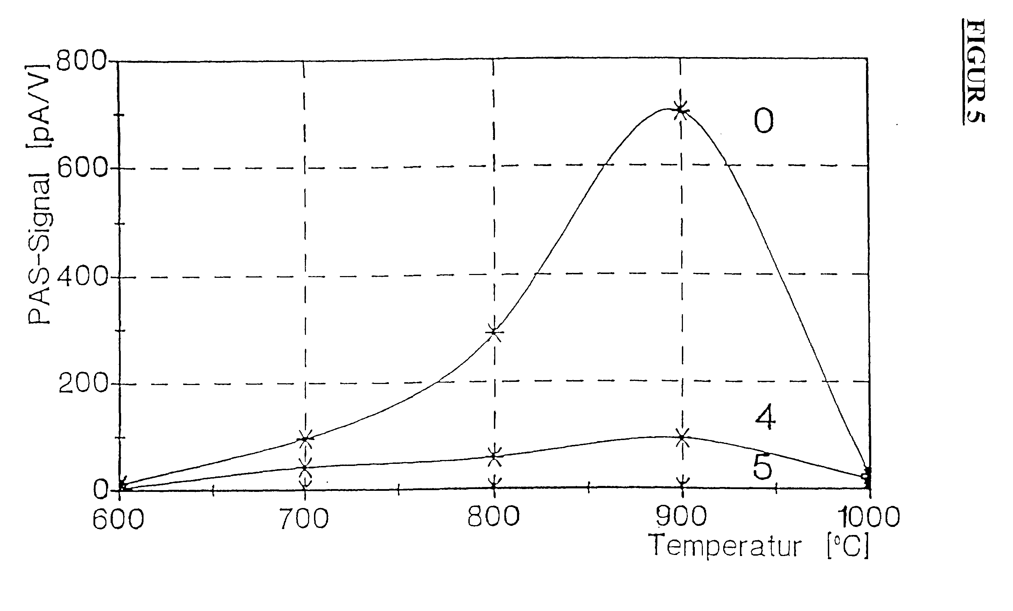

Figur 5 stellt Ergebnisse für qualitative Messungen an verschiedenen Proben dar.

Gemessen wurden benutzte Gießereisande, die unterschiedlich viele (0 und 4 bzw. 5) Umläufe in einer Aufbereitungsanlage erfahren hatten.

Für 0- bzw. 4fach aufbereiteten Sand wurde bei 900°C ein Signalmaximum gemessen. 5fach aufbereiteter Sand erzeugte nahezu kein Signal.

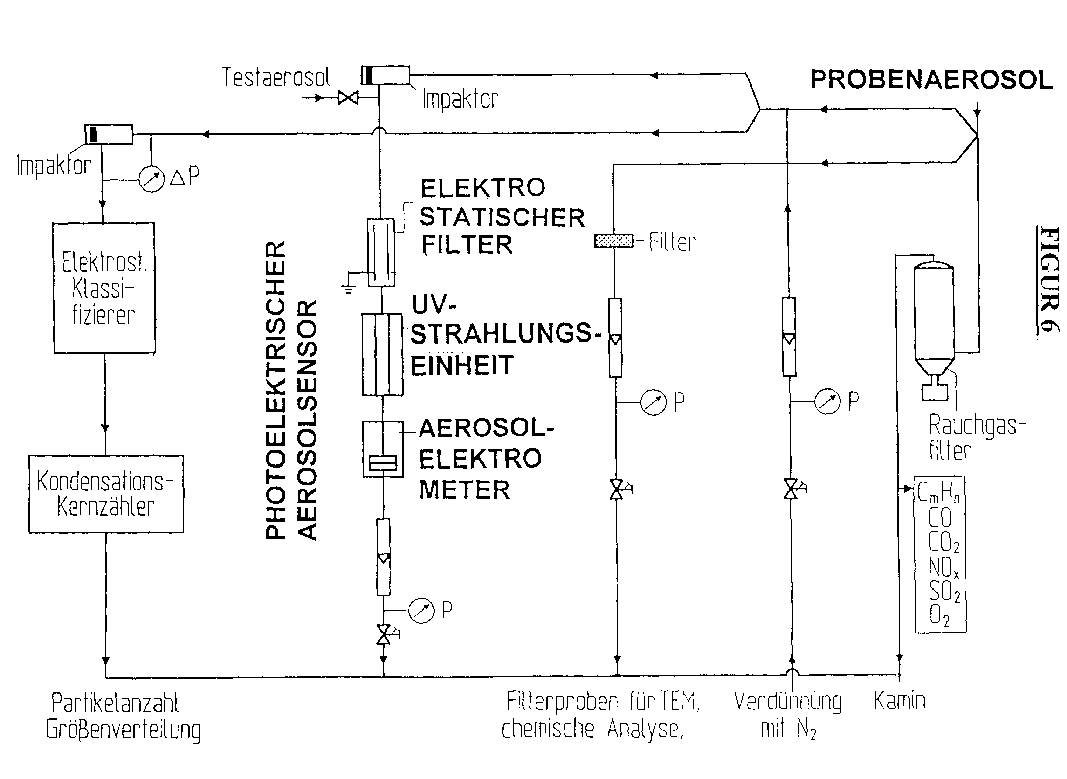

Figur 6 beschreibt die für die Untersuchungen zur Bestimmung des Gehaltes von in einer Probe befindlichem Material eingesetzte Anordnung. Bestandteile der Meßeinrichtung für das Probenaerosol sind
elektrostatische Filter,
UV-Bestrahlungseinheit,
Aerosolelektrometer.

Bei dieser Anordnung konnten folgende Messungen parallel erfolgen:
1) Messung der mittels UV-Bestrahlungseinheit erzeugten photoelektrische Aufladung des strömenden Probenaerosols.
   Die Messung der Partikelladung erfolgte mit einem Photoelektrischen-Aerosol-Sensor (PAS).
2) Prüfung der Korrelation des PAS-Signals und der PAH-Konzentration des Abgasaerosols durch naßchemische GC-Bestimmung des PAH-Gehaltes über Filterproben aus dem Abgas.
3) Prüfung der Oberflächenkorrelation durch Messung der Korngrößenverteilung mit elektrostatischem Klassierer und Kondensationspartikelzähler.
4) Sichtbarmachung der Mikrostrukturen der signalerzeugenden Partikel durch Darstellung mittels Transmissions-Elektronen-Mikroskop (TEM).
   Die Partikel werden durch elektrostatische Abscheidung auf mit Kohlenstoff beschichteten Standard-TEM-Netzen gesammelt.

Daten des für die Untersuchunqen benutzten Ofens (Kapillarofen):

| | |
|---|---|
| Länge des Ofens | 600 mm |
| Ofendurchmesser (innen) | 5,4 mm |
| Trägermedium Luftdurchfluß | ca. 0,6 m³ |
| Verweildauer | ca. 0,125 s |
| Unterdruck | 15 mbar |

angegebene Temperaturen sind Spitzenwerte

Kohlenstaub und andere Stoffe (ca. 400 mg/h, Teilchengröße > 300 nm) wurden in Luft dispergiert, erhitzt und analysiert.

### Untersuchungsergebnisse:

Die Experimente fanden bei Spitzentemperaturen zwischen 600 und 1000°C statt.
Vor der Analyse des Verbrennungsaerosols wurde das Abgas abgekühlt und mit Luft (1 : 3) und N₂ (1 : 5) verdünnt; dies entspricht einer absoluten Verdünnung von 1 : 15.
Luftdurchsatz, Verdünnungsverhältnis und UV-Lichtintensität wurden geeignet gewählt, um möglichst einfache Abhängigkeiten zwischen PAS-Signal, PAH-Konzentration und Probenstoffinhalt, bei zufriedenstellender Empfindlichkeit, zu erhalten.

Ein für verschiedene Proben gültiges Ergebnis betrifft die Größenverteilung der Partikel:

Für verschiedene Proben sind bei ungefähr 800°C, der Temperatur des maximalen PAS-Signals, sowohl der (aerodynamische) Durchmesser der Teilchen als auch die Konzentration der Teilchenanzahl maximal.
Wenn die Temperatur auf 1000°C gesteigert wird, verschieben sich die Korngrößenverteilung und die Anzahlkonzentration nur geringfügig. Das zugehörige PAS-Signal reduziert sich jedoch um mehr als 75 %. Dieses Ergebnis stimmt gut mit den naßchemisch bestimmten PAH-Konzentrationen überein. Bei niedrigeren Temperaturen (< 800°C) werden weniger und kleinere Agglomerate erzeugt, und das PAS-Signal nimmt weiter ab.
TEM-Aufnahmen zeigen, daß es sich bei allen Temperaturen um Agglomerate handelt, die im wesentlichen aus Primärkügelchen mit Durchmessern kleiner 50 nm bestehen. Diese bilden sich vermutlich in der Abkühlphase.

## Patentansprüche

1. Verfahren zur quantitativen Bestimmung von in einer Probe befindlichem Material mit den Schritten:
a)durch Thermobehandlung wird die Probe in mit photoemissionsfähige Stoffe bedeckte Submikronteilchen umgewandelt und so in einen photoemissionsfähigen Zustand gebracht,
b)in diesem photoemissionsfähigen Zustand wird sie der Photoemissionsmessung zugeführt,
c)anhand des bei dieser Photoemissionsmessung erhaltenen Meßsignals wird der Anteil des Materials in der Probe bestimmt,
d)die Bestimmung des Anteils des Materials in der Probe geschieht unter Zuhilfenahme von Meßdaten einer das Material in vorbestimmter Menge enthaltenden Vergleichsprobe oder unter Verwendung von durch Kalibrieren für das Material ermittelten Daten.

2. Verfahren nach Anspruch 1,
**dadurch gekennzeichnet,**
daß der Probe vor oder während der Thermobehandlung zur Erzeugung des photoemissionsfähigen Zustandes zur Verdünnung ein nichtemissionsfähiger, inerter Feststoff in geeigneter Form zugeführt wird.

3. Verfahren nach Anspruch 1 oder 2,
**dadurch gekennzeichnet,**
daß der Probe vor oder während der Thermobehandlung inerter Feststoff als zusätzlicher Trägerstoff zugefügt wird.

4. Verfahren nach Anspruch 1 bis 3,
**dadurch gekennzeichnet,**
daß der Probe ein zusätzliches Trägergas beigemischt wird.

5. Verfahren nach Anspruch 4,
**dadurch gekennzeichnet,**
daß das zusätzliche Trägergas ein Inertgas ist.

6. Verfahren nach Anspruch 1 bis 5,
**dadurch gekennzeichnet,**
daß das Material der Probe der Photoemissionsmessung kontinuierlich zugeführt und ein entsprechend kontinuierliches Meßsignal erhalten wird.

7. Verfahren nach einem der vorhergehenden Ansprüche,
**dadurch gekennzeichnet,**
daß für den Fall, daß sich in der Probe mehr als ein photoemissionsfähiges Material befindet, die Probe oder ein Teil der Probe in unterschiedlichen photoemissionsfähigen Zuständen der Photoemissionsmessung zugeführt werden und bei der quantitativen Bestimmung der Materialien die Meßdaten entsprechender Vergleichsproben oder entsprechende, durch Kalibrierung ermittelte Daten berücksichtigt werden.

8. Verfahren nach Anspruch 1 bis 7,
**dadurch gekennzeichnet,**
daß das Material ein organisches Material ist und der die Submikronteilchen bedeckende photoemissionsfähige Stoff polyaromatische Kohlenwasserstoffe sind.

9. Verfahren nach Anspruch 8,
**dadurch gekennzeichnet,**
daß die Probe auf eine Temperatur im Bereich von 600°C bis 1000°C erhitzt wird.

10. Verfahren nach Anspruch 9,
**dadurch gekennzeichnet,**
daß die Probe auf eine Temperatur im Bereich zwischen 750°C bis 850°C erhitzt wird.

11. Verfahren nach Anspruch 6, wahlweise in Verbindung mit den Maßnahmen gemäß den Ansprüchen 7, 9 oder 10,
**dadurch gekennzeichnet,**
daß das Material ein organisches, brennbares Material ist und das kontinuierliche Meßsignal als Führungsgröße zur Steuerung oder Regelung eines Verbrennungsprozesses eingesetzt wird.

12. Verfahren nach einem der Ansprüche 1 bis 10, dadurch gekennzeichnet, daß das Ergebnis der Bestimmung des Materials zur Überprüfung der Qualität der Probe, beispielsweise von Lebensmittelproben, eingesetzt wird.

13. Vorrichtung zur Durchführung des Verfahrens gemäß Anspruch 1, gekennzeichnet durch einen, ein elektrisches Meßsignal abgebenden Photoemissionssensor und eine, dem Sensor vorgeschaltete Einheit zur Erzeugung von photoemissionsfähigem Material durch Thermobehandlung und durch eine, dem Sensor nachgeschaltete elektronische Einrichtung, die anhand des Meßsignals den Anteil des Materials in der Probe unter Zuhilfenahme von Meßdaten einer das Material in vorbestimmter Menqe enthaltenden Vergleichsprobe oder unter Verwendung von durch Kalibrieren für das Material ermittelte Daten bestimmt und den ermittelten Wert anzeigt oder als digitalen oder elektrischen Wert ausgibt.

14. Vorrichtung nach Anspruch 13,
**dadurch gekennzeichnet,**
daß die dem Sensor vorgeschaltete Einheit eine Einheit zur Thermobehandlung zum Zwecke der Erzielung des photoemissionsfähigen Zustandes des Materials der Probe ist.

15. Vorrichtung nach Anspruch 14,
**dadurch gekennzeichnet,**
daß die Einheit zur Thermobehandlung auf eine vorgegebene Temperatur im Bereich zwischen 600°C und 1000°C einstellbar ist.

16. Vorrichtung nach Anspruch 15,
**dadurch gekennzeichnet,**
daß der Einheit zur Thermobehandlung eine Einheit zur Einspeisung von nichtemissionsfähigem, inertem Feststoff vorgeschaltet ist.

17. Vorrichtung nach einem der Ansprüche 13 bis 16,
**dadurch gekennzeichnet,**
daß dem Photoemissionssensor zusätzlich eine Einheit zum Einführen von zusätzlichem Trägergas vorgeschaltet ist.

18. Vorrichtung nach einem der Ansprüche 13 bis 17,
**dadurch gekennzeichnet,**
daß eine Einrichtung zur kontinuierlichen Zufuhr der Probe vorgesehen ist und die elektronische Einheit zur kontinuierlichen Abgabe des Meßsignals ausgelegt ist.

19. Vorrichtung nach Anspruch 18,
**dadurch gekennzeichnet,**
daß der elektronischen Einrichtung eine Steuer oder Regeleinrichtung nachgeschaltet ist.

## Claims

1. A method for the quantitative determination of material in a sample, comprising the steps:
a) the sample is converted by thermal treatment into submicron particles coated with photoemissive substances and is thus brought into a photoemissive state.
b) it is fed in this photoemissive state to the photoemission measurement,
c) the fraction of the material in the sample is determined by means of the measuring signal obtained during this photoemission measurement,
d) the determination of the fraction of the material in the sample is effected with the aid of measured data for a comparison sample containing the material in a predetermined amount or using data determined for the material by calibration.

2. A method according to claim 1,
characterised in that
a non-emissive, inert solid in a suitable form is added to the sample for dilution, before or during the thermal treatment for producing the photoemissive state.

3. A method according to claim 1 or 2,
characterised in that
an inert solid is added as an additional carrier substance to the sample before or during thermal treatment.

4. A method according to claims 1 to 3,
characterised in that
an additional carrier gas is admixed with the sample.

5. A method according to claim 4,
characterised in that
the additional carrier gas is an inert gas.

6. A method according to claims 1 to 5,
characterised in that
the material of the sample is continuously fed to the photoemissions measurement and a corresponding continuous measuring signal is obtained.

7. A method according to any one of the preceding claims,
characterised in that
if there is more than one photoemissive material in the sample, the sample or part of the sample is fed in different photoemissive states to the photoemission measurement, and measured data for corresponding comparison samples or corresponding data determined by calibration are taken into account during the quantitative determination of the materials.

8. A method according to claims 1 to 7,
characterised in that
the material is an organic material and the photoemissive substance which coats the submicron particles comprises polyaromatic hydrocarbons.

9. A method according to claim 8,
characterised in that
the sample is heated to a temperature within the range from 600°C to 1000°C.

10. A method according to claim 9,
characterised in that
the sample is heated to a temperature within the range between 750°C and 850°C.

11. A method according to claim 6, optionally in combination with the measures according to claims 7, 9 or 10,
characterised in that
the material is an organic, combustible material and the continuous measuring signal is used as a control input for controlling or regulating a combustion process.

12. A method according to any one of claims 1 to 10,
characterised in that
the result of the determination of the material is used for checking the quality of the sample, of foodstuff samples for example.

13. An apparatus for carrying out the method according to claim 1, characterised by a photoemission sensor which emits an electrical measuring signal and a unit connected upstream of the sensor for producing photoemissive material by thermal treatment, and by an electronic device, which is connected downstream of the sensor and which determines the fraction of the material in the sample by means of the measuring signal with the aid of measured data of a comparison sample containing the material in a predetermined amount or using data determined by calibration for the material, and which indicates the determined value or emits it as a digital or electrical value.

14. An apparatus according to claim 13,
characterised in that
the unit connected upstream of the sensor is a unit for thermal treatment for the purpose of attaining the photoemissive state of the material of the sample.

15. An apparatus according to claim 14,
characterised in that
the unit for thermal treatment can be set to a predetermined temperature within the range between 600°C and 1000°C.

16. An apparatus according to claim 15,
characterised in that
a unit for supplying non-emissive, inert solid is connected upstream of the unit for thermal treatment.

17. An apparatus according to any one of claims 13 to 16,
characterised in that
a unit for introducing additional carrier gas is additionally connected upstream of the photoemission sensor.

18. An apparatus according to any one of claims 13 to 17,
characterised in that
a device is provided for continuously feeding the sample, and the electronic unit is designed for the continuous output of the measuring signal.

19. An apparatus according to claim 18,
characterised in that
a controlling or regulating device is connected upstream of the electronic device.

## Revendications

1. Procédé de détermination quantitative d'une substance se trouvant dans un échantillon comprenant les stades:
a) l'échantillon est transformé par traitement thermique en particules submicroniques recouvertes d'une matière apte à la photoémission et est mis ainsi en un état apte à la photoémission,
b) il est envoyé en cet état apte à la photoémission à la mesure de la photoémission,
c) au moyen du signal de mesure obtenu dans cette mesure de la photoémission, la proportion de la substance dans l'échantillon est déterminée,
d) la détermination de la proportion de la substance dans l'échantillon s'effectue à l'aide de données de mesure d'un échantillon de comparaison contenant la substance en une quantité déterminée à l'avance ou en utilisant des données déterminées pour la substance par étalonnage.

2. Procédé suivant la revendication 1,
caractérisé
en ce qu'il est ajouté à l'échantillon avant le traitement thermique d'obtention de l'état susceptible de la photoémission pour une dilution une matière solide inerte sous forme appropriée, qui n'est pas susceptible de donner une émission.

3. Procédé suivant la revendication 1 ou 2,
caractérisé
en ce qu'il est ajouté à l'échantillon avant le traitement thermique ou pendant le traitement thermique une matière solide inerte comme matière porteuse supplémentaire.

4. Procédé suivant l'une des revendications 1 à 3,
caractérisé
en ce qu'il est mélangé à l'échantillon un gaz porteur supplémentaire.

5. Procédé suivant la revendication 4,
caractérisé
en ce que le gaz porteur supplémentaire est un gaz inerte.

6. Procédé suivant la revendication 1 à 5,
caractérisé
en ce que la substance de l'échantillon est ajoutée en continu à la mesure de photoémission et il est obtenu de manière correspondante un signal de mesure continu.

7. Procédé suivant l'une des revendications précédentes,
caractérisé
en ce que dans le cas où il se trouve dans l'échantillon plus d'une substance susceptible de la photoémission, l'échantillon ou une partie de l'échantillon est envoyé à la mesure de la photoémission en des états susceptibles de photoémissions qui sont différents et lors de la détermination quantitative des substances, les données de mesure d'échantillons de comparaison correspondants ou les données correspondantes déterminées par étalonnage sont prises en compte.

8. Procédé suivant l'une des revendications 1 à 7,
caractérisé
en ce que la substance est une matière organique et la matière susceptible de photoémission recouvrant les particules submicroniques est un hydrocarbure polyaromatique.

9. Procédé suivant la revendication 8,
caractérisé
en ce que l'échantillon est porté à une température de l'ordre de 600°C à 1000°C.

10. Procédé suivant la revendication 9,
caractérisé en ce que l'échantillon est porté à une température de l'ordre de 750°C à 850°C.

11. Procédé suivant la revendication 6, éventuellement en liaison avec les dispositions suivant les revendications 7,9 ou 10,
caractérisé
en ce que la substance est une substance organique combustible et le signal de mesure continu est utilisé comme grandeur de pilotage pour commander ou pour réguler un processus de combustion.

12. Procédé suivant l'une des revendications 1 à 10,
caractérisé
en ce que le résultat de la détermination de la substance est utilisé pour vérifier la qualité de l'échantillon, par exemple d'échantillons de denrées alimentaires.

13. Installation pour la mise en oeuvre du procédé suivant la revendication 1, caractérisée par un détecteur de photoémission émettant un signal électrique de mesure et, en amont du détecteur, une unité de production de matière apte à photoémettre par traitement thermique et, en aval du détecteur, un dispositif électronique qui, au moyen du signal de mesure, indique la proportion de la substance dans l'échantillon à l'aide de données de mesure d'un échantillon de comparaison contenant la substance en une quantité déterminée à l'avance ou en utilisant des données déterminées pour la substance par étalonnage et indique la valeur déterminée ou donne une valeur numérique ou électrique.

14. Installation suivant la revendication 13,
caractérisée
en ce que l'unité montée en amont du détecteur est une unité de traitement thermique afin d'obtenir l'état susceptible de la photoémission de la substance de l'échantillon.

15. Installation suivant la revendication 14,
caractérisée
en ce que l'unité de traitement thermique peut être réglée à une température donnée à l'avance dans l'intervalle compris entre 600°C et 1000°C.

16. Installation suivant la revendication 15,
caractérisée
en ce qu'il est monté, en amont de l'unité de traitement thermique, une unité d'alimentation en matière solide inerte, non susceptible de donner lieu à une émission.

17. Installation suivant l'une des revendications 13 à 16,
caractérisée
en ce qu'il est monté, en amont du détecteur de la photoémission, en plus une unité d'injection de gaz porteur supplémentaire.

18. Installation suivant l'une des revendications 13 à 17,
caractérisée
en ce qu'il est prévu un dispositif d'envoi continu de l'échantillon et l'unité électronique est conçue pour donner en continu le signal de mesure.

19. Installation suivant la revendication 18,
caractérisée
en ce qu'il est monté, en aval du dispositif électronique, une commande ou un dispositif de régulation.
